# EUROPEAN PATENT APPLICATION

(11) **EP 3 695 813 A1**
(43) Date of publication of application: **19.08.2020**
(21) Application number: 19163564.8
(22) Date of filing: 18.03.2019
(51) Int. Cl.: A61F 2/58, A61F 2/42

(54) **ACTIVE PROSTHETIC WRIST**

(30) Priority: 14.02.2019 EP 19461512
(71) Applicant: VBIONIC Sp. z o.o., 60-612 Poznan (PL)
(72) Inventor: Rajewski, Bartosz, 60-612 Poznan (PL); Luczak, Adam, 61-674 Poznan (PL)
(74) Representative: Kancelaria Eupatent.pl Sp. z.o.o

(57) **Abstract**

A method for operating an active prosthetic wrist (10) comprising an attached prosthetic hand (12), the method comprising the steps of: awaiting (501) a placement of the prosthetic hand (12) around an object and setting an initial position of the prosthetic hand (12) in space; defining (502) a reference surface as a plane perpendicular to the vector of center of gravity; awaiting (503) reception of an activation signal; Awaiting (504) reception of a signal informing that said object has been seized by the user using the active prosthetic wrist (10); setting up a reference position (505) of the entire prosthesis (10, 12); Monitoring (506), by means of sensors (403), rotation along at least one axis; converting the rotation signals into information on position in relation to said reference surface, of the reference position, and maintaining (507) a vector of center of gravity with respect to the previously stor

## Description

### TECHNICAL FIELD

The present invention relates to a system and a method for an active prosthetic wrist. In particular, the present invention relates to an active prosthetic wrist having improved capabilities of grabbing, lifting, moving, steady holding etc. of vessels filled with liquids.

### BACKGROUND OF THE INVENTION

Active prosthetic wrists are used in upper limb prosthesis, operating as a connection between a prosthetic hand and a prosthetic socket on the residual part of the forearm, and in case of the amputation of the arm- the elbow or enucleation of the shoulder joint, it shall be a connection with a part of a prosthesis representing the forearm.

Most upper limb prostheses are equipped with wrists that can only rotate, either passively by using the non-amputated hand, or actively through a myoelectric signal. A myoelectric signal, also called a motor action potential, is an electrical impulse that produces contraction of muscle fibers in the body.

The present invention is aimed at aiding stabilization of everyday activities such as, for example, carrying objects, and, in particular, lifting and moving dishes containing liquid content. Moreover, the present invention is to provide support for the residual limb or its absence- with a proper prosthesis on, by higher control over movements. In the end, functionality of a prosthetic wrists as well as comfort of use shall be improved leading to greater satisfaction of a user.

Prior art defines a Modular prosthesis (MPL) of John Hopkins University Applied Physics Laboratory (see: https://www.ihuapl.edu/prosthetics/scientists/mpl.asp) having a prosthetic wrist capable of effectuating a tri-axial rotation, listed in order, starting from the closer position: pronation and supination, that is external and internal rotation; abduction and adduction; flexion and extension.

An axis of rotation of each activator is situated at the angle of 90° to the two remaining activators.

A disadvantage of such solution is lack of stabilization in two axes as well as lack of ability to use the wrist as a separate prosthetic component. The aim of the development of the present invention is active stabilization of a prosthetic wrist.

### SUMMARY AND OBJECTS OF THE PRESENT INVENTION

A first object of the present invention is a method for operating an active prosthetic wrist comprising an attached prosthetic hand the method comprising the steps of: awaiting a placement of the prosthetic hand around an object and setting an initial position of the prosthetic hand in space; defining a reference surface as a plane perpendicular to the vector of center of gravity; awaiting reception of an activation signal; Awaiting reception of a signal informing that said object has been seized by the user using the active prosthetic wrist; setting up a reference position of the entire prosthesis; Monitoring, by means of sensors, rotation along at least one axis; converting the rotation signals into information on position in relation to said reference surface, of the reference position, and maintaining a vector of center of gravity with respect to the previously stored position.

Preferably, said initial and reference positions are previously predefined and are initiated by a user.

Preferably, said activation signal is provided by means of a switch button or a voice command.

Preferably, said monitoring and maintaining is effected in three axes. Preferably, said maintaining step is configured for stabilization in the xy plane, which results in stabilization in the transverse plane.

Another object of the present invention is an active prosthetic wrist comprising: a prosthetic wrist; a power source; and a motors unit comprising at least one motor configured to move the prosthetic wrist, said active prosthetic wrist being characterized in that it further comprises: a sensors unit comprising a tri-axial accelerometer; an activation/deactivation means configured to receive a signal from a user; a controller configured to execute all steps of the method according to the present invention.

Preferably, said active prosthetic wrist is connected to a prosthetic hand.

Preferably, an effector responsible for flexion and extension of the prosthetic hand is positioned closer to a prosthesis socket than an effector responsible for pronation and supination.

Preferably, said sensors unit comprises a tri-axial gyroscope or/and a tri-axial magnetometer.

Preferably, said prosthetic wrist is a separate prosthetic component.

Preferably, said motors unit comprises effectors that are brushless, electric motors with harmonic drives.

Preferably, said activation/deactivation means is a button or a touch-detector or a voice detector or a tap detector.

Preferably, the active prosthetic wrist further comprises a current sensor configured to determine an overload condition.

Yet another object of the present invention is a computer program comprising program code means for performing all the steps of the computer-implemented method according to the present invention when said program is run on a computer.

A further object of the present invention is a computer readable medium storing computer-executable instructions performing all the steps of the computer-implemented method according to the present invention when executed on a computer.

### BRIEF DESCRIPTION OF THE DRAWINGS

These and other objects of the invention presented herein, are accomplished by providing a system and method for an active prosthetic wrist. Further details and features of the present invention, its nature and various advantages will become more apparent from the following detailed description of the preferred embodiments shown in a drawing, in which:
Fig. 1 presents a basic overview of the elements of the of the active prosthetic wrist according to the present invention;
Fig. 2 depicts an example of a mode of operation of the present invention;
Fig. 3 shows examples of operation of a prosthetic wrist stabilization along two axes;
Fig. 4 presents a block diagram of the system according to the present invention; and
Fig. 5 shows a presents a block diagram of the method according to the present invention.

### NOTATION AND NOMENCLATURE

Some portions of the detailed description which follows are presented in terms of data processing procedures, steps or other symbolic representations of operations on data bits that can be performed on computer memory. Therefore, a computer executes such logical steps thus requiring physical manipulations of physical quantities.

Usually these quantities take the form of electrical or magnetic signals capable of being stored, transferred, combined, compared, and otherwise manipulated in a computer system. For reasons of common usage, these signals are referred to as bits, packets, messages, values, elements, symbols, characters, terms, numbers, or the like.

Additionally, all of these and similar terms are to be associated with the appropriate physical quantities and are merely convenient labels applied to these quantities. Terms such as "processing" or "creating" or "transferring" or "executing" or "determining" or "detecting" or "obtaining" or "selecting" or "calculating" or "generating" or the like, refer to the action and processes of a computer system that manipulates and transforms data represented as physical (electronic) quantities within the computer's registers and memories into other data similarly represented as physical quantities within the memories or registers or other such information storage.

A computer-readable (storage) medium, such as referred to herein, typically may be non-transitory and/or comprise a non-transitory device. In this context, a non-transitory storage medium may include a device that may be tangible, meaning that the device has a concrete physical form, although the device may change its physical state. Thus, for example, non-transitory refers to a device remaining tangible despite a change in state.

As utilized herein, the term "example" means serving as a non-limiting example, instance, or illustration. As utilized herein, the terms "for example" and "e.g." introduce a list of one or more non-limiting examples, instances, or illustrations.

### DESCRIPTION OF EMBODIMENTS

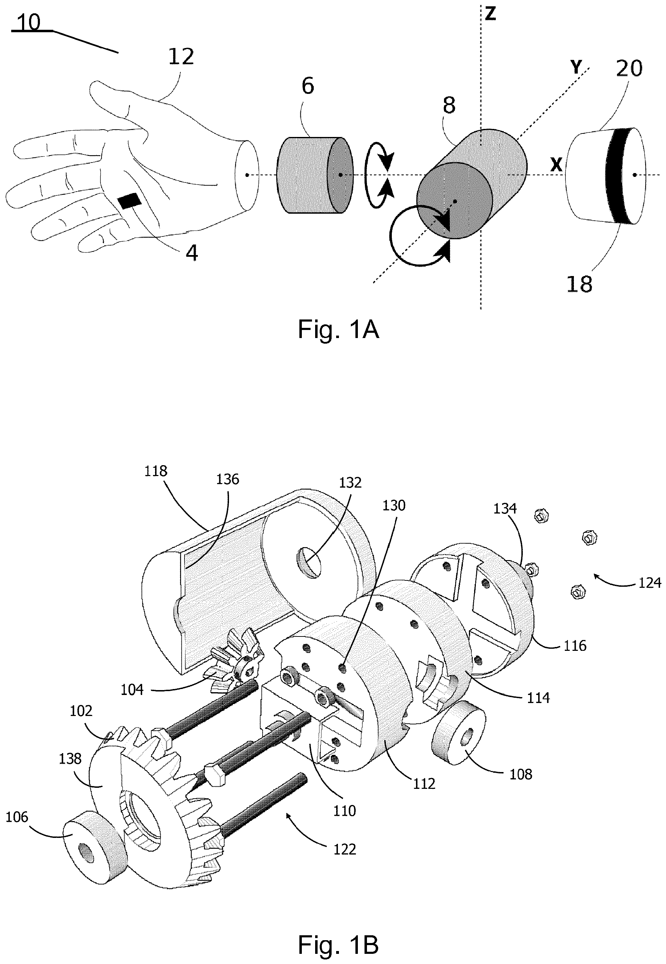

Fig. 1A presents a basic overview of the elements of the of the active prosthetic wrist 10 according to the present invention.

The active prosthetic wrist 10, is based mainly on stabilization at least along one axis, favourably along two axes, where said two axes concern flexion and extension as well as pronation and supination of a properly functioning limb.

The active prosthetic wrist 10 has at least two active degrees-of-freedom (DOF), corresponding to pronation/supination, flexion/extension and optionally it may have a third degree corresponding to adduction/abduction joints.

Extension occurs when the angle between two adjacent segments of the body increases and occurs in a sagittal plan about a frontal axis. Correspondingly, flexion occurs when the angle between two adjacent segments of the body decreases and occurs in a sagittal plane about a frontal axis.

Said stabilization may concern the third axis as well, that is shifting abduction and adduction of a wrist, of a properly functioning limb, to movement of the prosthetic wrist 10.

Stabilization of a prosthetic hand 12 by said prosthetic wrist 10 comprises blocking rotation changes of the prosthetic hand 12 at least along one axis in relation to a reference surface. It allows, for example, for moving safely a dish, filled with a liquid, from one place to another without spilling the content of said dish.

The main source of data necessary for said stabilization is a sensor 4 comprising a tri-axial accelerometer. Additionally, the data from a tri-axial gyroscope or/and a tri-axial magnetometer may be used. Favourably, the sensor 4 is a Bosch Sensortec GmbH BMO055 sensor comprising an accelerometer, a gyroscope and a magnetometer.

Data, at least from the accelerometer, are processed in a control unit 18 by means of an adequate algorithm into information on tri-axial position in relation to the surface.

Said control unit may be positioned in an adapter 20, enabling the connection with prosthetic sockets available on the market.

Once processed in a control unit 18, the processed data are used to control motors in order to compensate for even the tiniest tilts (for example tilts below 1 degree).

The location of the sensor 4 may be a surface or structure of the prosthetic hand 12 as well as the surface of the prosthetic wrist 10 itself in order to make it possible to use the prosthetic wrist 10 as a separate prosthetic unit capable of being connected with other prosthetic components.

Favourably, the operation of the present invention, as a part of the upper limb prosthesis, may be activated or deactivated when necessary. In a situation, in which according to a user, stabilization of a prosthetic hand 12 by means of the prosthetic wrist 10 is required, it can be activated in a simple way by an initiating signal generated by means of a button, a voice command or muscle tension.

After detecting the signal, the device is activated and stabilizes the attached prosthetic hand 12 at least along one axis, favourably along two axes. Deactivation takes place by means of a different or the same initiating signal when a user decides there is not any need for further use of the stabilizing function or in other situation. Deactivation can be automatic as well, for example after detection that a held object has been released.

Said initiating signals may be pre-defined and stored in a memory. A particular initiating signal is assigned to activate stabilization and a particular deactivating signal is assigned to deactivation or it can be as well a list of signals to choose from for a user- users can choose an initiating and/ or deactivating signal themselves or such an initiating signal can be programmable for a particular user and a user can program an initiating signal themselves.

Fig. 1A additionally presents placement of effectors (for example said effectors are brushless electric motors with harmonic drives.) in the prosthetic wrist 10. The closest, taking the anatomical aspects into consideration, there is the adapter 20, enabling a connection with prosthetic sockets available on the market. The control unit 18 responsible for controlling and supplying the components with power is preferably placed in the adapter as well.

Next there is an effector 8 and favourably it is a brushless DC motor with a harmonic drive, whose function is flexion and extension of the prosthetic hand, that is rotation along y axis. An effector 6 follows, and preferably it is also a brushless DC motor with a harmonic drive, whose function is pronation and supination, namely rotation along the x axis. The system, in which rotations take place in said axes, allows for stabilization in the xy plane, which results in stabilization in the transverse plane for the adducted upper limb. Optionally, rotations in the z plane may be supported and operate similarly to rotations in the x and y planes.

The aforementioned elements 4, 6, 8, 18 and 20 may be integrated in one housing.

Fig. 1B presents a more detailed version of the general overview of Fig. 1A. A prosthetic wrist is shown having core segments 110, 112, 114 and 116 arrangement on one axis in the aforementioned order wherein the core segment is facing the prosthetic hand while the core segments 116 is facing the limb. The core segments 112, 114 and 116 are preferably shaped in a form of cylinders (or substantially cylindrical shapes) wherein their bases face each other and are in planes perpendicular to the aforementioned one axis arrangement.

The engine mounting segments 110 allows for mounting an engine to the core segment 112 with appropriate screws. The engine mounted between the core segments 110 and 112 drives a toothed wheel 104 (preferably a bevel gear), which in turn drives a second gear wheel 102 (preferably a bevel gear operating at 90 degrees angle with respect to the toothed wheel 104). The second gear wheel 102 is mounted on a pin 111 (positioned on the same axis as the core segments) of the core segment 110 and a bearing 106 fitting inside the second gear wheel 102 in its center.

Between the core segments 112 and 114 there is positioned a second electric engine having its effector mounted in a bearing 108. The axis of the bearing 108 is perpendicular to the axis of positioning the bearing 106 as well as the axis of the core segments 110, 112, 114 and 116.

Between the core segments 114 and 116 there is preferably positioned a control panel (not shown) allowing a user control of the system.

The core segments 112,114 and 116 are connected to each other by means of screws 122 via screw holes present on the perimeter of each cylinder. One of such screw holes is a screw hole 130 which. The screw mounting has suitable nuts such as hexagonal nuts.

The outer base of the core element 116 may comprises recesses for receiving the corresponding nuts 124, which allows for easier mounting and better fitting an outer cover 118.

The outer cover 118 is mounted on the toothed wheel 102 by a glued or screw mount wherein a section 136, of the toothed wheel 102, adheres to the section 138 of the outer cover 118. On the other end, the outer cover 118 is fastened to the core segment 116 by a bearing 134 mounted in a radial opening 132 positioned in the base of the outer cover 118 coinciding with the base of the core segments 116.

The outer cover 118 is mounted to a forearm, while a screw supported on the bearing 108 is connected to the prosthetic hand. The section 138 will not move but the internal parts will move i.e. the core segments 112, 114 and 116, which will result in turning the hand that may be mounted on a screw running via the bearing 108.

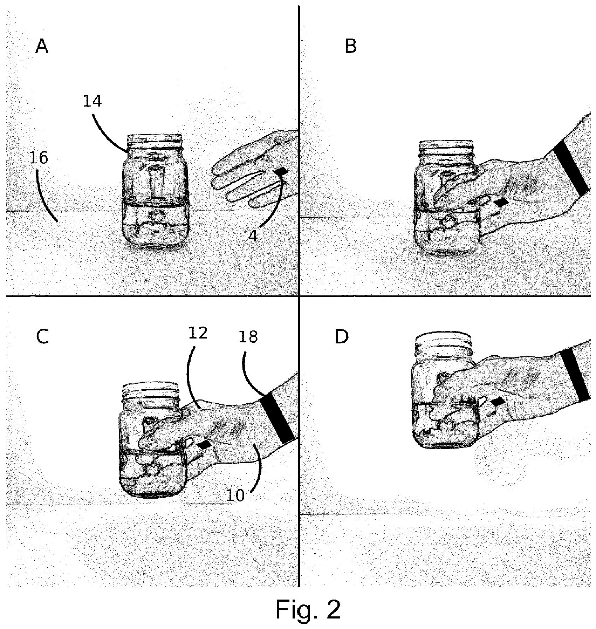

An example of a mode of operation of the present invention has been shown in Fig. 2. A user of the upper limb prosthesis 12 comprising the prosthetic wrist 10 is able to use stabilization, after a preceding activation. Said stabilization is effected as follows in four steps A - D:
A - a user wishes to move a dish 14 containing a liquid and therefore places the prosthesis 12 around it;
B - next the user seizes said dish 14. Activation of stabilization takes place by means of an initiating signal. At the moment of activation, the position of the prosthesis 12 is saved on the basis of the data from the position sensor 4 comprising the accelerometer and optionally the gyroscope and/ or the magnetometer;
C - At the moment of lifting the dish 14, the sensor 4 constantly monitors rotation along the three axes and appropriately transmits signals to the control unit 18;
D - the signals are converted, in the control unit 18, into the information on position in relation to the surface 16. Said information on position is related to maintaining a vector of center of gravity (this may be based on for example known technique described in the following publication: Hasnain, B. A., & Algoz, A. (2018). A Control System For A 3-Axis Camera Stabilizer (Dissertation). Retrieved from http://urn.kb.se/resolve?urn=urn:nbn:se:uu:diva-355845, p. 7.).

In case of detection of tilting, that is rotation along one of the axes, a signal correction is sent to motors and in turn to the effector 6 and the effector 8 placed in the prosthetic wrist 10, in order to correct said detected tilting. Owing to that process, any tilting is corrected immediately and the constant inclination angles of the prosthetic hand 12 in relation to the surface 16 are maintained. Everything works in real time and at the sampling rate sufficient to hold the preset position steady.

The surface 16 is defined as a plane perpendicular to the vector of center of gravity.

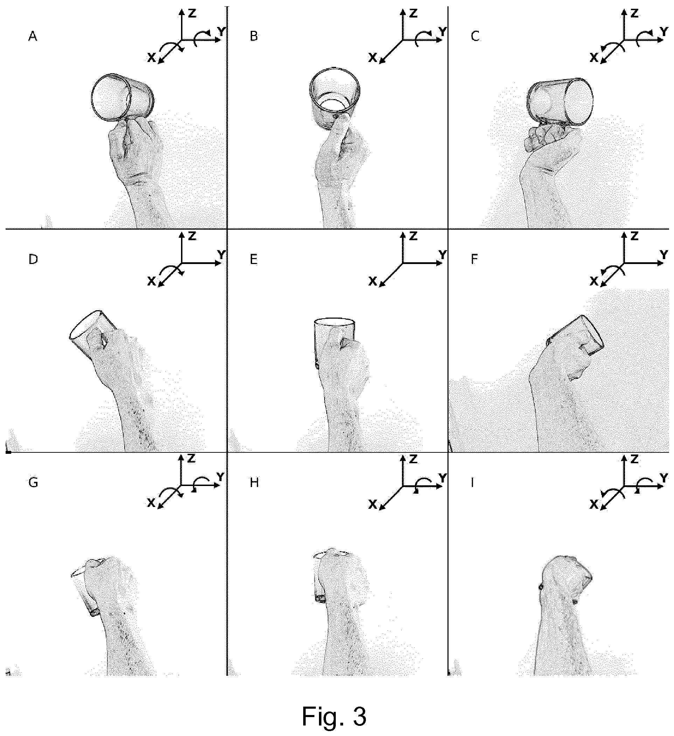

An example of operation of stabilization along two axes is presented below. Stabilization may take place along two axes, which is presented in Fig. 3. Nine use cases, which require a different effect as movements of motors, of the prosthetic wrist 10, are shown.

The use cases shown in pictures A, C, G and I, require motor rotations along two axes in various combinations of rotation directions. The use cases B, D, F and H require an action of one motor only, in which rotation takes place in one of two directions. The last use case E does not require any compensation. Directions of rotation compensation for each use case are presented in the right upper corner of each and every subpicture A - I. The axes requiring compensation are determined as well as the directions of rotation of each effector.

The compensation is effected such that values of acceleration vectors (preferably in 3 axes or in each of the axes considered) are kept as constant as possible by adjusting engines that results in positioning of the prosthesis. After each parameters reading a correction of prosthesis 12 is made and vectors values are read again and compared to reference values.

The X axis is the one passing through the axis of rotation of the prosthetic wrist 10 and compensation along the X axis is responsible for pronation or supination.

The Y axis is the one passing through the prosthetic wrist and compensation along the Y axis is responsible for flexion and extension.

The situations illustrated above are only a schematic representation. In reality the tilts are not visible as the motors react to slight tilts with high frequency (for example Bosch BNO055 is capable of 400 Hz sampling and is a System in Package (SiP), integrating a triaxial 14-bit accelerometer, a triaxial 16-bit gyroscope with a range of ±2000 degrees per second, a triaxial geomagnetic sensor and a 32-bit micro-controller), which results in compensation of very small angles in relation to the originally saved position.

The system of two effectors 6 and 8, the sensor 4 and the control unit 18 can also be employed as a system allowing for placing the prosthetic hand 12 in a position required to perform a particular action. It can be based on classification of the system of gestures. Each gesture is based on the data from the sensor 4, which results in rotation in relation to the surface along two axes in the effector 6 and effector 8, that is placing the upper limb in space.

Initiation of a gesture setting could be based on a voice signal, muscle tension or other. After initiating a gesture, by means of the data from the sensor 4, the control unit 18 would send the signals to the effector 6 and effector 8, which would result in setting the expected, earlier defined position of the effectors 6, 8. The position of the effectors 6, 8 is a particular angle or angles in relation to the surface 16. After setting a gesture, it is possible to maintain the angles in relation to the surface or blocking the position of motors.

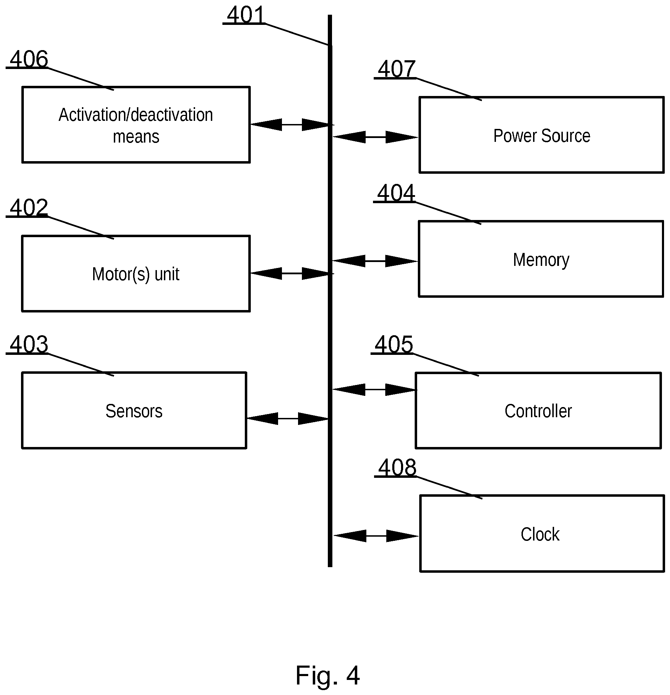

Fig. 4 presents a diagram of the system according to the present invention. The system is integrated in a prosthesis and controls its movements.

The system may be realized using dedicated components or custom made FPGA (field-programmable gate array) or ASIC (application-specific integrated circuit) circuits or a combination thereof. The system comprises a data bus 401 communicatively coupled to a memory 404. Additionally, other components of the system are communicatively coupled to the system bus 401 so that they may be managed by a controller 405, 18.

The memory 404 may store computer program or programs executed by the controller 405 in order to execute steps of the method according to the present invention. System configuration may also be stored in said memory 404.

A motors unit 402 comprises at least one motor controlled by the controller 405. The motor unit may comprise its own controller and suitable encoders, effectors 6, 8. The motors unit 402 is preferably connected to the controller 18 in a wired manner.

A sensors unit 403 comprises the aforementioned sensor 4 and is controlled by said controller 405 in response to a predefined stabilization plane as well as current readings of the sensor 4.

The sensors unit 403 may also optionally comprise a current sensor configured to determine an overload condition (for example an object has been lifted which is outside allowed maximum weight)
Activation/deactivation means 406, is any system that may receive a signal from a user, this may be a button, a touch-detector, a voice detector, a tap detector or the like.

A clock module 407 is configured to provide a suitable sampling rate trigger for sampling data from said sensors unit 403. Preferably, the sampling rate is in a range of 1-100 MHz and preferably around 30 MHz.

A power source module 408 is preferably a battery powered power supply that powers all components of the system.

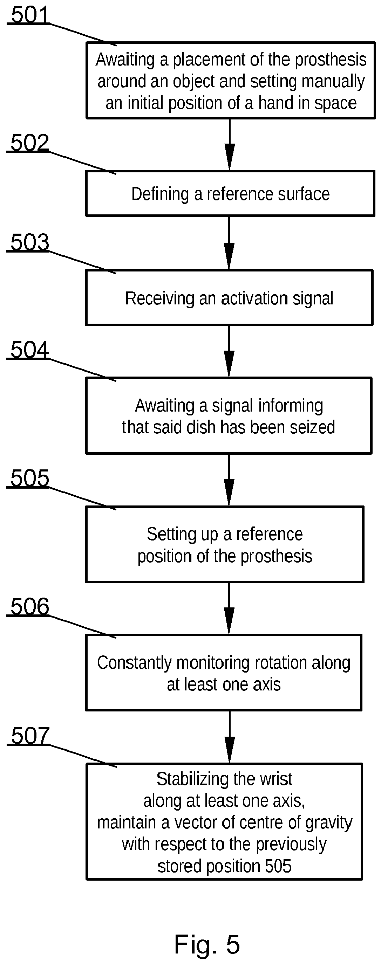

Fig. 5 presents a diagram of the method according to the present invention. The method applies to the prosthetic wrist shown in the preceding figures. It starts at step 501 from awaiting a placement of the prosthesis around an object and setting manually (in some embodiments the initial position may be set using hardware means such as electric engine, gear, actuator i.e. using the active prosthetic wrist and hand) an initial position of the hand in space. In practice this means a placement of the prosthesis 12 around an object such as a dish or said dish's handle.

Next, at step 502, a reference surface is defined as a plane perpendicular to the vector of centre of gravity.

Further, at step 503, the process awaits receiving an activation signal, typically from a user using a switch button or a voice command.
Subsequently, at step 504, the process awaits a signal informing that said dish has been seized by the user. This sets up a reference position 505 of the entire prosthesis 12.

At step 506 the sensors 403 constantly monitor rotation along the three axes (at least one axis and preferably three axes) and appropriately transmits signals to the control unit 18.

Lastly, the signals are converted, in the control unit 18, into the information on position in relation to the surface 16. Said information on position is related to maintaining a vector of centre of gravity with respect to the previously stored position 505.

Optionally, a user may define, and program in memory of the device, the most frequently used hand prosthesis 12 positions. These positions are defined with reference to a surface and may later be requested by an activation signal selecting one of these predefined positions. In such cases the process of Fig. 5 is modified to use a predefined position.

One of the most important aspect of the present invention is a stabilization of a prosthetic wrist that in turn moves a prosthetic hand 12. The stabilization is switched on demand to a given reference position which shall be maintained irrespective of the users arm movements.

The prosthetic wrist, according to the present invention, is used as a mechanical device to operate a prosthetic hand whereas depending on needs, a stabilization feature may be switched on.

The present invention defines a physical device of an active prosthetic wrist, facilitating movements of a prosthetic hand. Further, some tasks may be performed quicker and with less difficulty to a user as a result of wrist flexibility and stabilization. Therefore, the invention provides a useful, concrete and tangible result.

The specification above defines a machine as well as processing of data received from appropriate sensors of the machine. Thus, the machine or transformation test is fulfilled and that the idea is not abstract.

At least parts of the methods according to the invention may be computer implemented. Accordingly, the present invention may take the form of an entirely hardware embodiment, an entirely software embodiment (including firmware, resident software, micro-code, etc.) or an embodiment combining software and hardware aspects that may all generally be referred to herein as a "circuit", "module" or "system".

Furthermore, the present invention may take the form of a computer program product embodied in any tangible medium of expression having computer usable program code embodied in the medium.

It can be easily recognized, by one skilled in the art, that the aforementioned method for an active prosthetic wrist may be performed and/or controlled by one or more computer programs. Such computer programs are typically executed by utilizing the computing resources in a computing device. Applications are stored on a non-transitory medium. An example of a non-transitory medium is a non-volatile memory, for example a flash memory while an example of a volatile memory is RAM. The computer instructions are executed by a processor. These memories are exemplary recording media for storing computer programs comprising computer-executable instructions performing all the steps of the computer-implemented method according the technical concept presented herein.

While the invention presented herein has been depicted, described, and has been defined with reference to particular preferred embodiments, such references and examples of implementation in the foregoing specification do not imply any limitation on the invention. It will, however, be evident that various modifications and changes may be made thereto without departing from the broader scope of the technical concept. The presented preferred embodiments are exemplary only, and are not exhaustive of the scope of the technical concept presented herein.

Accordingly, the scope of protection is not limited to the preferred embodiments described in the specification, but is only limited by the claims that follow.

## Claims

1. A method for operating an active prosthetic wrist (10) comprising an attached prosthetic hand (12), the method comprising the steps of:
- awaiting (501) a placement of the prosthetic hand (12) around an object and setting an initial position of the prosthetic hand (12) in space;
- defining (502) a reference surface (16) as a plane perpendicular to the vector of center of gravity;
- awaiting (503) reception of an activation signal;
- awaiting (504) reception of a signal informing that said object has been seized by the user using the active prosthetic wrist (10);
- setting up a reference position (505) of the entire prosthesis (10, 12);
- monitoring (506), by means of sensors (403), rotation along at least one axis;
- converting the rotation signals into information on position in relation to said reference surface (16), of the reference position; and
- maintaining (507) a vector of center of gravity with respect to the previously stored position (505).

2. The method according to claim 1 wherein said initial and reference positions are previously predefined and are initiated by a user.

3. The method according to any of previous claims, wherein said activation signal is provided by means of a switch button or a voice command.

4. The method according to any of previous claims, wherein said monitoring (506) and maintaining (507) is effected in three axes.

5. The method according to any of previous claims, wherein said maintaining (507) step is configured for stabilization in the xy plane, which results in stabilization in the transverse plane.

6. An active prosthetic wrist comprising:
- a prosthetic wrist (10);
- a power source (407); and
- a motors unit (402) comprising at least one motor configured to move the prosthetic wrist (10),
said active prosthetic wrist being **characterized in that** it further comprises:
- a sensors unit (403) comprising a tri-axial accelerometer;
- an activation/deactivation means (406) configured to receive a signal from a user; and
- a controller (405) configured to execute all steps of the method according to any of claims 1-5.

7. The active prosthetic wrist of claim 6 wherein said active prosthetic wrist is connected to a prosthetic hand (12).

8. The active prosthetic wrist of claim 7 wherein an effector (8) responsible for flexion and extension of the prosthetic hand (12) is positioned closer to a prosthesis socket than an effector (6) responsible for pronation and supination.

9. The active prosthetic wrist of any of claims 6-8 wherein said sensors unit (403) comprises a tri-axial gyroscope or/and a tri-axial magnetometer.

10. The active prosthetic wrist of any of claims 6-9 wherein said prosthetic wrist is a separate prosthetic component.

11. The active prosthetic wrist of any of claims 6-10 wherein said motors unit (402) comprises effectors that are brushless, electric motors with harmonic drives.

12. The active prosthetic wrist of any of claims 6-11 wherein said activation/deactivation means (406) is a button or a touch-detector or a voice detector or a tap detector.

13. The active prosthetic wrist of any of claims 6-12 further comprising a current sensor configured to determine an overload condition.

14. A computer program comprising program code means for performing all the steps of the computer-implemented method according to any of claims 1-5 when said program is run on a computer.

15. A computer readable medium storing computer-executable instructions performing all the steps of the computer-implemented method according to any of claims 1-5 when executed on a computer.
